# FASCICULE DE BREVET EUROPEEN

(11) **EP 0 618 181 B1**
(45) Date de publication et mention de la délivrance du brevet: **27.08.1997**
(21) Numéro de dépôt: 94200758.4
(22) Date de dépôt: 24.03.1994
(51) Int. Cl.: C07C 19/08, C07C 21/18, C07C 17/38

(54) **Compositions azéotropiques comprenant de l'eau et un hydrofluorocarbure, procédé d'élimination d'eau d'une solution par distillation azéotropique et procédé de production d'un hydrofluoroalcane**
Azeotrope bestehend aus Wasser und aus einem Fluorkohlenwasserstoff, Verfahren zur Entfernung von Wasser aus einer Lösung durch Azeotrop destillation und Verfahren zur Herstellung eines Fluorkohlenwasserstoffs
Azeotropic compositions comprising water and a hydrofluoroalkane, process for the elimination of water from a solution by a zeotropic distillation and process for the preparation of a hydrofluoroalkane

(30) Priorité: 01.04.1993 BE 9300321
(43) Date de publication de la demande: 05.10.1994
(73) Titulaire: SOLVAY (Société Anonyme), B-1050 Bruxelles (BE)
(72) Inventeur: Balthasart, Dominique, B-1120 Bruxelles (BE); Jacquemart, André, B-1310 La Hulpe (BE)
(74) Mandataire: Jacques, Philippe

(56) Documents cités:
- DD-A- 83 984
- US-A- 3 275 549
- Advances in Chemistry, Vol. 116, Lee H. Horsley: Azeotropic Data-III, American Chemical Society, Washington D.C., 1973, pages 615-628

## Description

L'invention concerne un procédé d'élimination d'eau d'une solution par distillation azéotropique et un procédé de production d'un hydrofluoroalcane.

Il est connu de préparer des hydrofluoroalcanes par réaction d'hydrocarbures halogénés, saturés ou non, avec du fluorure d'hydrogène. Dans ce procédé connu, est obtenu un mélange de produits réactionnels comprenant l'hydrofluoroalcane recherché et du fluorure d'hydrogène n'ayant pas réagi, ainsi que, habituellement, du chlorure d'hydrogène et divers composés organiques halogénés, notamment la fraction de l'hydrocarbure halogéné mis en oeuvre n'ayant pas réagi ainsi que divers sous-produits. Il est non seulement nécessaire de pouvoir alors isoler, sous une forme substantiellement pure, l'hydrofluoroalcane recherché mais il est aussi primordial pour l'économie du procédé de pouvoir recycler le fluorure d'hydrogène et tout autre réactif non converti au réacteur de fluoration.

Dans les procédés de synthèse d'hydrofluoroalcanes, le fractionnement du mélange de produits réactionnels en ses différents constituants est effectuée, au moins en partie, par distillation. A titre d'exemples, on peut citer les documents EP-A-0003147 (SOLVAY) et EP-A-0098341 (PENNWALT) relatifs à la scission du mélange de produits réactionnels obtenus dans le cadre de la synthèse du 1-chloro-1,1-difluoroéthane par réaction entre du fluorure d'hydrogène et du chlorure de vinylidène ou du 1,1,1-trichloroéthane. On peut aussi mentionner la demande de brevet EP-A-0467531 (I.C.I.) relative à la scission par distillation du mélange de produits réactionnels obtenu dans le cadre de la synthèse du 1,1,1,2-tétrafluoroéthane par réaction entre du fluorure d'hydrogène et du trichloroéthylène ou du 2-chloro-1,1,1-trifluoroéthane.

Dans les procédés de préparation d'hydrofluoroalcanes par réaction avec du fluorure d'hydrogène, la présence d'eau est généralement préjudiciable à la bonne marche du procédé. Outre un éventuel effet négatif sur les performances de la synthèse de l'hydrofluoroalcane recherché, l'accroissement de la teneur en eau dans le mélange de produits réactionnels provoque inévitablement une corrosion accrue des appareillages. En pratique, la mise en oeuvre de réactifs parfaitement anhydres s'avère cependant très difficile. Or, il est maintenant apparu, qu'en raison de la forte affinité du fluorure d'hydrogène pour l'eau, en cas de recyclage des réactifs non consommés au réacteur de synthèse, l'eau accompagne généralement le fluorure d'hydrogène recyclé, ce qui provoque une augmentation de la teneur en eau dans la boucle "réacteur - unité de séparation et recyclage du fluorure d'hydrogène". Il apparaît dès lors nécessaire d'éliminer l'eau présente dans le mélange de produits réactionnels avant recyclage.

Le brevet DD-83984 divulgue un procédé d'élimination d'eau de tétrafluorodibromoéthane brut par addition de méthanol au tétrafluorodibromoéthane brut et distillation d'un mélange azéotropique ternaire constitué de tétrafluorodibromoéthane, de méthanol et d'eau. Un tel procédé implique obligatoirement la présence de tétrafluorodibromoéthane et de méthanol dans le mélange à sécher.

Par ailleurs, dans certains procédés de préparation d'hydrofluoroalcanes, les hydrocarbures insaturés, présents à titres d'impuretés dans le mélange de produits réactionnels, sont difficilement séparables par distillation de l'hydrofluoroalcane recherché, en raison de leur température d'ébullition trop voisine de celle dudit hydrofluoroalcane et sont dès lors transformés par chloration en hydrocarbures saturés plus lourds, lesquels peuvent alors être facilement séparés dudit hydrofluoroalcane. Une telle opération de chloration implique la mise en contact du mélange de produits réactionnels, éventuellement déjà débarrassé de certains de ses constituants, avec du chlore, introduit généralement en excès par rapport aux hydrocarbures insaturés à transformer. On a maintenant observé que, dans certaines conditions de fonctionnement, l'eau présente dans le mélange de produits réactionnels ou amenée avec le chlore forme avec le chlore des hydrates Cl₂ - H₂O. Ces hydrates peuvent s'accumuler sous forme solide dans les zones froides de l'installation, par exemple dans le condenseur en tête de la colonne de distillation destinée à séparer, du produit réactionnel, le Cl₂ excédentaire en vue de son recyclage. Lors des arrêts de l'installation, ces hydrates risquent de se décomposer en raison du réchauffement des zones froides de l'installation et de former une solution particulièrement agressive. Outre les problèmes aigus de corrosion qu'ils engendrent, les hydrates de chlore risquent également d'obstruer les appareillages et d'en limiter ainsi la capacité de production.

L'élimination d'eau de compositions liquides par distillation azéotropique est bien connue et est décrite, par exemple, dans "Encyclopedia of Chemical Technology", 1978, 3ème édition, volume 3, p. 361-373. Selon ce procédé, on ajoute à la composition un entraîneur, qui est un composé formant un azéotrope avec l'eau, et on chauffe et/ou on détend ensuite la solution résultante pour vaporiser l'azéotrope. Lorsque la solution est soumise à une distillation, l'azéotrope eau/entraîneur est éliminé en tête de la colonne de distillation.

On a maintenant trouvé que des hydrofluorocarbures bien définis forment des azéotropes avec l'eau, ce qui permet, dans certaines conditions, l'élimination d'eau présente dans diverses solutions, en particulier dans des mélanges de produits réactionnels recueillis lors de la production d'hydrofluoroalcanes, évitant ainsi les inconvénients sus-mentionnés lors de la production d'hydrofluoroalcanes.

Forment des azéotropes avec l'eau, le 1,1-dichloro-1-fluoroéthane, le 1-chloro-1,1-difluoroéthane, le 1,1-difluoroéthane, le 1,1,1-trifluoroéthane, le 1,1,1,2-tétrafluoroéthane et le trifluoroéthylène.

Fondamentalement, l'état thermodynamique d'un fluide est défini par quatre variables interdépendantes : la pression (P), la température (T), la composition de la phase liquide (X) et la composition de la phase gazeuse (Y). Un azéotrope vrai est un système particulier à 2 ou plusieurs composants pour lequel, à une température donnée et à une pression donnée, X est exactement égal à Y. Un pseudo-azéotrope est un système à 2 ou plusieurs composants pour lequel, à une température donnée et à une pression donnée, X est substantiellement égal à Y. En pratique, cela signifie que les constituants de tels systèmes azéotropiques et pseudo-azéotropiques sont sensiblement inséparables par distillation.

Aux fins de la présente invention, on entend, par composition azéotropique, un mélange de deux ou plusieurs constituants, qui présente les propriétés d'un azéotrope vrai ou d'un pseudo-azéotrope, pour lequel le rapport molaire des constituants du mélange diffère de celui de l'azéotrope vrai de 10 % au maximum, ou dont le point d'ébullition (à une pression donnée) diffère du point d'ébullition de l'azéotrope vrai de 0,5 °C au maximum, ou encore dont la tension de vapeur (à une température donnée) diffère de celle de l'azéotrope vrai de 10 mbar au maximum. On sait que, dans un azéotrope, la concentration des constituants, la température et la tension de vapeur sont des paramètres interdépendants. Dès lors que l'un de ces paramètres est fixé, les autres sont imposés.

Dans la gamme habituelle de pressions mises en oeuvre dans les procédés classiques de production d'hydrofluoroalcanes, à savoir de 0,1 à 100 bar, la teneur en eau dans les compositions azéotropiques aux fins de l'invention est au moins égale à 0,01 % molaire et au plus égale à 10 % molaire. Le plus souvent, cette teneur est supérieure ou égale à 0,1 % molaire. De préférence, elle est inférieure ou égale à 5 % molaire.

A 20 °C, la composition azéotropique constituée essentiellement de 1,1-dichloro-1-fluoroéthane (HFA-141b) et d'eau contient ces constituants dans un rapport molaire eau : HFA-141b égal à (4 ± 0,4) : 100. A cette température, la tension de vapeur de la composition azéotropique constituée essentiellement de HFA-141b et d'eau est de 0,67 ± 0,01 bar.

A 20 °C, la composition azéotropique constituée essentiellement de 1-chloro-1,1-difluoroéthane (HFA-142b) et d'eau contient ces constituants dans un rapport molaire eau : HFA-142b égal à (1,1 ± 0,1) : 100. A cette température, la tension de vapeur de la composition azéotropique constituée essentiellement de HFA-141b et d'eau est de 2,93 ± 0,01 bar.

A 20 °C, la composition azéotropique constituée essentiellement de 1,1,1-trifluoroéthane (HFA-143a) et d'eau contient ces constituants dans un rapport molaire eau : HFA-143a égal à (0,3 ± 0,03) : 100. A cette température, la tension de vapeur de la composition azéotropique constituée essentiellement de HFA-143a et d'eau est de 11 ± 0,01 bar.

L'invention n'exploite pas seulement les compositions particulières définies ci-dessus. Elle vise l'exploitation de toute composition azéotropique constituée essentiellement d'eau et d'au moins un hydrofluorocarbure sélectionné parmi le 1,1-dichloro-1-fluoroéthane, le 1-chloro-1,1-difluoroéthane, le 1,1-difluoroéthane, le 1,1,1-trifluoroéthane, le 1,1,1,2-tétrafluoroéthane et le trifluoroéthylène.

Les compositions azéotropiques ci-dessus trouvent une application dans les procédés d'élimination d'eau, par distillation azéotropique, de solutions comprenant de l'eau et au moins un composé liquide autre que l'eau, telles que notamment les mélanges de produits réactionnels obtenus lors de la production d'hydrofluoroalcanes.

L'invention concerne dès lors un procédé d'élimination d'eau d'une solution comprenant de l'eau et au moins un composé liquide autre que de l'eau, par distillation azéotropique d'un azéotrope eau/entraîneur, qui se caractérise en ce que l'entraîneur est sélectionné parmi le 1,1-dichloro-1-fluoroéthane, le 1-chloro-1,1-difluoroéthane, le 1,1-difluoroéthane, le 1,1,1-trifluoroéthane, le 1,1,1,2-tétrafluoroéthane et le trifluoroéthylène.

Par entraîneur, on entend désigner un hydrofluorocarbure formant avec l'eau une composition azéotropique conforme à l'invention, laquelle composition peut être éliminée en tête d'une colonne de distillation lorsque la solution est soumise à une distillation.

Le procédé d'élimination d'eau selon la présente invention est particulièrement applicable aux solutions contenant de l'eau en faible teneur, généralement inférieure à 30 % en poids. De préférence, la quantité d'eau de la solution ne dépasse pas 10 % du poids de la solution. Le plus souvent, cette quantité ne dépasse pas 5 %. Elle est généralement d'au moins 5 ppm en poids. Elle est le plus souvent d'au moins 50 ppm en poids.

Dans le procédé selon l'invention, on choisit généralement comme entraîneur un hydrofluorocarbure formant avec l'eau un azéotrope dont le point d'ébullition est inférieur au point d'ébullition du composé autre que l'eau, présent dans la solution, de telle sorte que l'azéotrope eau/entraîneur soit éliminé à l'état de vapeur lors de la distillation. Dans une variante du procédé appliquée au cas où la solution comprend certains composés présentant un point d'ébullition inférieur à celui de l'azéotrope formé par l'eau et l'entraîneur, ces composés sont séparés de la solution avec l'azéotrope.

Dans le procédé d'élimination d'eau selon l'invention, on soumet la solution contenant l'eau à éliminer à une étape de distillation en présence d'une quantité suffisante d'entraîneur.

La quantité nécessaire d'entraîneur dépend de différents paramètres, tels que notamment sa nature, la teneur résiduelle en eau visée dans la solution, la pression et la température auxquelles est réalisée la distillation et la nature et la proportion des composés de la solution contenant l'eau à éliminer. Il est normalement souhaitable que l'entraîneur soit présent en quantité suffisante pour former un azéotrope avec pratiquement toute l'eau contenue dans la solution. En règle générale, la quantité d'entraîneur mise en oeuvre est de 1 à 10 fois la quantité d'entraîneur strictement nécessaire pour former l'azéotrope avec la totalité de l'eau présente dans la solution. De préférence, la quantité d'entraîneur est de 1,2 à 5 fois ladite quantité strictement nécessaire. De manière particulièrement préférée, la quantité d'entraîneur est de 1,5 à 3 fois ladite quantité strictement nécessaire.

Dans le procédé d'élimination d'eau selon l'invention, le composé liquide autre que l'eau, cité plus haut, n'est pas critique et peut être indifféremment un composé organique ou un composé inorganique. Il peut notamment comprendre des hydracides (par exemple du fluorure d'hydrogène ou du chlorure d'hydrogène) ou des hydrocarbures, éventuellement substitués, notamment par des halogènes (par exemple des hydrofluoroalcanes).

Le procédé d'élimination d'eau selon l'invention est avantageux pour éliminer l'eau de solutions comprenant un hydrofluoroalcane comme composé liquide autre que l'eau. Il est particulièrement avantageux pour éliminer l'eau de solutions dans lesquelles le composé liquide autre que l'eau constitue, en partie au moins, l'entraîneur formant un azéotrope avec l'eau. Dans cette application de l'invention, les solutions contenant un hydrofluoroalcane peuvent être des compositions diverses, telles que des compositions de nettoyage, de dégraisssage ou de séchage d'objets, tels que, par exemple, des pièces de mécanique fine ou des pièces d'optique.

Le procédé d'élimination d'eau selon l'invention est tout particulièrement avantageux pour éliminer l'eau de mélanges de produits réactionnels obtenus lors de la production d'un hydrofluoroalcane.

L'invention concerne dès lors également un procédé de production d'un hydrofluoroalcane dans lequel on recueille un mélange de produits réactionnels comprenant, en solution, l'hydrofluoroalcane et de l'eau, on soumet ledit mélange à une distillation azéotropique en présence d'un entraîneur formant un azéotrope avec l'eau, et on recueille de la distillation un distillat contenant l'azéotrope et une phase liquide contenant l'hydrofluoroalcane, le procédé se caractérisant en ce qu'on sélectionne l'entraîneur parmi le 1,1-dichloro-1-fluoroéthane, le 1-chloro-1,1-difluoroéthane, le 1,1-difluoroéthane, le 1,1,1-trifluoroéthane, le 1,1,1,2-tétrafluoroéthane et le trifluoroéthylène.

Le procédé selon l'invention de production d'un hydrofluoroalcane s'applique à la production de tout hydrofluoroalcane dont le point d'ébullition est supérieur à celui de l'azéotrope précité. Il s'applique notamment à la production d'hydrofluoroalcanes comprenant de 2 à 6 atomes de carbone et répondant à la formule générale CₐH_{b}F_{c}X_{d} dans laquelle X désigne Cl et/ou Br, de préférence Cl, a est un nombre entier de 2 à 6, b est un nombre entier de 1 à 13, c est un nombre entier de 1 à 13 et d est un nombre entier de 0 à 8, avec b + c + d = 2a + 2 lorsque l'hydrofluoroalcane est acyclique et avec b + c + d = 2a lorsque l'hydrofluoroalcane est alicyclique. Il s'applique particulièrement à la production d'hydrofluoroalcanes acycliques répondant à la formule générale ci-dessus dans laquelle X est Cl, a est un nombre entier égal à 2, 3 ou 4, b est un nombre entier de 1 à 9, c est un nombre entier de 1 à 9 et d est un nombre entier de 0 à 5. Il s'applique plus particulièrement à la production d'hydrofluoroalcanes acycliques répondant à la formule générale ci-dessus dans laquelle X est Cl, a est un nombre entier égal à 2 ou 3, b est un nombre entier de 1 à 6, c est un nombre entier de 1 à 6 et d est un nombre entier de 1 à 4. A titre d'exemples, le procédé selon l'invention est notamment applicable à la production des hydrofluoroalcanes de formule CH₃CCl₂F, CH₃CClF₂, CH₃CHF₂, CH₃CF₃, CH₂FCH₂F, CH₂FCHF₂, CH₂FCF₃, CHF₂CCl₃, CHF₂CF₃, CHCl₂CF₃, CHF₂CHF₂, CF₃CHClF, CF₃CF₂CHCl₂, CF₂ClCF₂CHClF, CF₃CH₂CF₂CH₃ et CF₃CH₂CH₂CF₃. Le procédé selon l'invention est tout particulièrement applicable à la production de 1,1-dichloro-1-fluoroéthane.

Dans le procédé de production d'un hydrofluoroalcane selon l'invention, l'hydrofluoroalcane est généralement préparé par réaction d'un halogène, d'un hydracide ou d'hydrogène avec un hydrocarbure halogéné saturé ou insaturé. A titre d'exemples de telles réactions, on peut citer la synthèse du 1,1-dichloro-1-fluoroéthane et/ou du 1-chloro-1,1-difluoroéthane par hydrofluoration du chlorure de vinylidène ou du 1,1,1-trichloroéthane. On peut aussi mentionner la synthèse du 1,1,1,2-tétrafluoroéthane par hydrofluoration catalytique d'un composé de formule CX₃CH₂Cl ou CX₂=CHX, avec X égal à Cl ou F. Les conditions opératoires dans lesquelles ces réactions sont mises en oeuvre sont bien connues dans l'art antérieur.

Par mélange de produits réactionnels, on entend tout milieu liquide comprenant, à l'état dissous, de l'eau, l'hydrofluoroalcane à produire et, éventuellement, des substances additionnelles telles que, par exemple, des réactifs non convertis, des sous-produits et des impuretés. Il peut être indifféremment un mélange brut, recueilli immédiatement à l'issue de la synthèse, définie plus haut, de l'hydrofluoroalcane et contenant des substances additionnelles, ou un mélange épuré au moins en partie desdites substances additionnelles.

Généralement, à côté de l'hydrofluoroalcane recherché, le mélange de produits réactionnels contient d'autres hydrofluoroalcanes sous-produits dans la synthèse ou, lorsque l'hydrofluoroalcane recherché est obtenu au départ d'un autre hydrofluoroalcane, une fraction non convertie de cet hydrofluoroalcane de départ. Il peut contenir au moins un hydracide, tel que le fluorure d'hydrogène ou le chlorure d'hydrogène. Il contient fréquemment du fluorure d'hydrogène. Il peut contenir un halogène, tel que le chlore ou le fluor. En outre, il contient généralement différents composés halogénés, pouvant être des réactifs non convertis dans l'étape de synthèse ou des sous-produits de celle-ci. Il peut aussi contenir, à l'état dissous, différents composés inorganiques, utilisés dans la synthèse, soit comme gaz inerte, tel l'azote, ou comme réactif, tel l'hydrogène.

La teneur en eau dans le mélange de produits réactionnels peut varier dans de larges mesures, tout en restant toutefois inférieure à la teneur en hydrofluoroalcane dans le mélange de produits réactionnels. Elle ne dépasse généralement pas 5 % du poids du mélange de produits réactionnels. Le procédé selon l'invention est particulièrement adapté à l'élimination d'eau présente en faibles quantités, par exemple lorsque la teneur en eau dans le mélange de produits réactionnels est comprise entre 5 et 5000 ppm en poids. Typiquement, le mélange de produits réactionnels contient de telles teneurs en eau lorsque les réactifs mis en oeuvre pour synthétiser l'hydrofluoroalcane renferment de l'ordre de 10 à 500 ppm d'eau.

Dans le procédé de production d'un hydrofluoroalcane selon l'invention, la distillation peut être réalisée dans toute colonne de distillation classique. L'azéotrope étant du type à point d'ébullition minimum, il est récupéré en tête de la colonne, dans le distillat. Le mélange de produits réactionnels, débarrassé de l'eau qu'il contenait est récupéré au pied de la colonne, dans la phase liquide. Cette phase liquide contient l'hydrofluoroalcane à produire. Elle comprend généralement aussi au moins certaines des substances additionnelles définies plus haut. Le cas échéant, pour séparer ces substances additionnelles de l'hydrofluoroalcane, on soumet la phase liquide de la distillation à une opération de séparation classique en ses constituants en vue d'isoler, sous une forme substantiellement pure, l'hydrofluoroalcane recherché. Les substances additionnelles séparées peuvent être recyclées de manière la plus appropriée, selon leur nature et leur rôle dans la synthèse de l'hydrofluoroalcane à produire. De préférence, la distillation de l'azéotrope eau/entraîneur est effectuée dans une colonne de distillation effectuant simultanément la séparation d'autres constituants du mélange de produits réactionnels ayant une volatilité proche de celle de l'azéotrope utilisé.

En règle générale, la quantité d'entraîneur présent dans le mélange de produits réactionnels que l'on soumet à la distillation est de 1 à 10 fois la quantité strictement nécessaire pour former l'azéotrope avec l'eau présente dans le mélange de produits réactionnels. De préférence, la quantité d'entraîneur présent dans le mélange de produits réactionnels est de 1,2 à 5 fois ladite quantité strictement nécessaire. De manière particulièrement préférée, la quantité d'entraîneur présent dans le mélange de produits réactionnels est de 1,5 à 3 fois ladite quantité strictement nécessaire.

Dans un premier mode d'exécution du procédé selon l'invention de production d'un hydrofluoroalcane, l'entraîneur mis en oeuvre est un constituant du mélange de produits réactionnels. Il peut s'agir d'un réactif n'ayant pas été entièrement converti dans la synthèse de l'hydrofluoroalcane recherché. Il peut s'agir d'un sous-produit formé lors de la synthèse. Il peut également s'agir de l'hydrofluoroalcane recherché. Dans ce dernier cas, on règle les conditions de la distillation de telle manière que seule la quantité d'hydrofluoroalcane nécessaire à l'élimination de l'eau soit recueillie dans le distillat, la quantité restante, de préférence majoritaire, de l'hydrofluoroalcane quittant la colonne de distillation dans la phase liquide de la distillation. Ce premier mode d'exécution du procédé selon l'invention présente l'avantage d'éviter tout recours à un composé extérieur au procédé. Il n'est cependant possible que lorsqu'un des constituants du mélange de produits réactionnels forme avec l'eau un azéotrope à point d'ébullition plus bas que celui de l'hydrofluoroalcane à produire et qu'il est présent dans le mélange en quantité suffisante pour permettre d'éliminer une quantité satisfaisante de l'eau contenue dans ce mélange.

Dans un second mode d'exécution du procédé selon l'invention de production d'un hydrofluoroalcane, on ajoute au mélange de produits réactionnels une partie au moins de l'entraîneur. Un tel mode d'exécution est nécessaire lorsque le mélange de produits réactionnels ne contient pas d'hydrofluorocarbures formant avec l'eau un azéotrope tel que défini plus haut, ou les contient en quantité insuffisante. On peut effectuer cet ajout dans le mélange réactionnel avant de le soumettre à la distillation ou introduire l'entraîneur directement dans la colonne de distillation.

Après distillation, l'azéotrope peut, le cas échéant, être séparé des autres constituants du distillat par toute méthode appropriée. Il est également possible de fractionner l'azéotrope eau/entraîneur en ses constituants, par exemple par séchage au moyen de substances dessicatives, ou encore par décantation lorsque l'azéotrope est un hétéroazéotrope, comme c'est le cas des compositions 1,1-dichloro-1-fluoroéthane/eau et 1-chloro-1,1-difluoroéthane/eau. Un tel fractionnement de l'azéotrope est souhaitable lorsque l'on veut récupérer l'hydrofluorocarbure mis en oeuvre comme entraîneur. Tel est notamment le cas lorsque l'hydrofluorocarbure est un réactif intervenant dans la synthèse de l'hydrofluoroalcane recherché ou un composé volontairement introduit dans le mélange de produits réactionnels en vue de former l'azéotrope avec l'eau. Il est alors de préférence recyclé dans l'installation de production de l'hydrofluoroalcane à l'endroit le plus approprié selon sa fonction. Lorsque l'entraîneur est l'hydrofluoroalcane recherché, selon que la fraction de l'hydrofluoroalcane éliminée dans le distillat est plus ou moins importante, on procède ou non à une étape de récupération de l'azéotrope eau/hydrofluoroalcane, suivie d'une étape de séparation de l'eau et de l'hydrofluoroalcane, afin de récupérer la fraction de l'hydrofluoroalcane passée dans le distillat.

L'invention va maintenant être illustrée en se référant aux figures annexées, lesquelles représentent, de manière schématique, le diagramme des flux de deux modes particuliers de mise en oeuvre du procédé selon l'invention de production d'un hydrofluoroalcane. Ces exemples sont donnés à titre illustratif et n'entendent pas limiter l'invention à ces applications particulières.

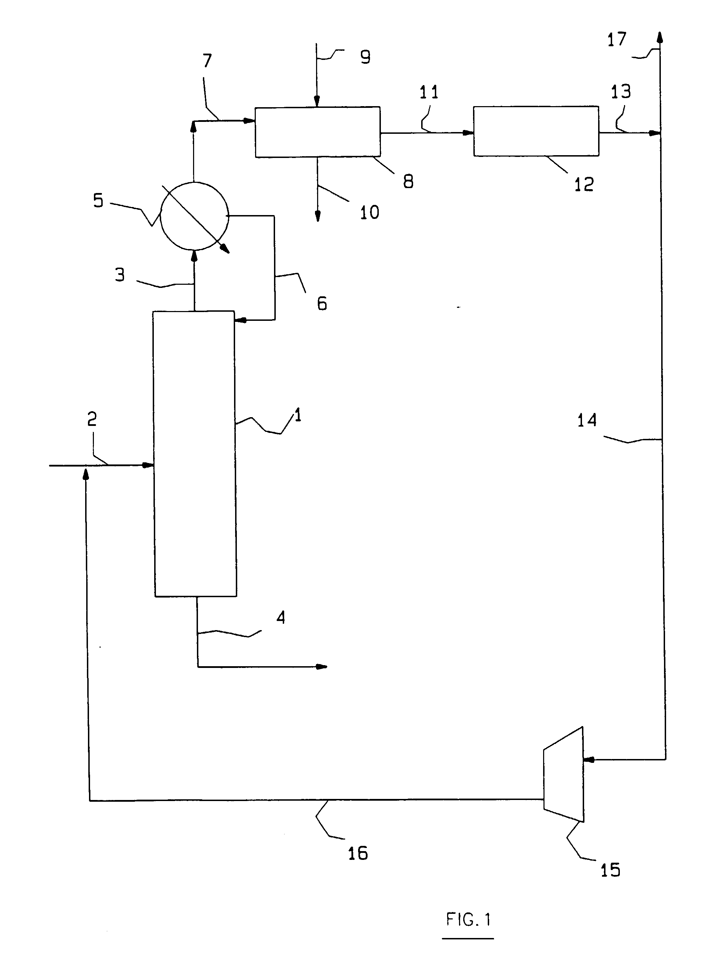

Le procédé schématisé à la figure 1 est destiné à la fabrication conjointe de 1,1-dichloro-1-fluoroéthane et de 1-chloro-1,1-difluoroéthane. A cet effet, on fait réagir, dans un réacteur non représenté, du fluorure d'hydrogène et du chlorure de vinylidène et on recueille du réacteur un mélange de produits réactionnels comprenant typiquement de 2,5 à 60 % molaire de 1,1-dichloro-1-fluoroéthane, de 2,5 à 60 % molaire de 1-chloro-1,1-difluoroéthane, de 30 à 90 % molaire de fluorure d'hydrogène, de 5 à 50 % molaire de chlorure d'hydrogène, de 50 à 250 ppm d'eau et, éventuellement, du 1,1,1-trifluoroéthane (jusqu'à 5 % molaire). Le mélange réactionnel, désigné par la notation de référence (2), est introduit sous forme liquide dans une colonne de distillation (1), avec, éventuellement, un complément de 1,1,1-trifluoroéthane à titre d'entraîneur tel que défini plus haut. Le complément de 1,1,1-trifluoroéthane est réglé de manière telle que, dans le mélange soumis à la distillation, le rapport molaire entre le flux de 1,1,1-trifluoroéthane et le flux de mélange réactionnel soit classiquement de 0,2 à 10 %. La colonne de distillation est maintenue à une pression de 8 à 20 bar. Le distillat quittant la colonne par le conduit (3) comprend principalement le chlorure d'hydrogène, l'azéotrope eau/1,1,1-trifluoroéthane et le 1,1,1-trifluoroéthane excédentaire éventuel. Il renferme en outre de l'azote. Après condensation partielle dans un condenseur (5), la partie condensée du distillat (6) est renvoyé à la colonne comme reflux et la partie non condensée du distillat (7) est envoyée dans une colonne d'absorption (8). Dans celle-ci, le chlorure d'hydrogène est séparé du distillat par absorption dans de l'eau introduite en (9). Le chlorure d'hydrogène et l'eau sont évacués par le conduit (10). Le flux gazeux résiduel (11) contenant principalement un mélange de 1,1,1-trifluoroéthane et de gaz inertes saturés en eau est ensuite amené dans un sécheur (12) pour éliminer l'eau. Dans le sécheur (12), le flux gazeux (11) peut par exemple traverser des lits de matière adsorbante. Le gaz (13), recueilli du sécheur (12) est constitué quasi exclusivement de 1,1,1-trifluoroéthane et de gaz inertes. Il est renvoyé via le conduit (14), le compresseur (15) et le conduit (16) à la colonne de distillation (1). Le conduit (17) constitue une purge, par laquelle sont évacués les gaz inertes tels que l'azote et, éventuellement, le 1,1,1-trifluoroéthane excédentaire. Au pied de la colonne de distillation, on recueille une phase liquide (4). Celle-ci contient classiquement le 1,1-dichloro-1-fluoroéthane et le 1-chloro-1,1-difluoroéthane coproduits, le chlorure de vinylidène et le fluorure d'hydrogène non réagis et moins de 10 ppm d'eau. Cette phase liquide peut être fractionnée en ses différents constituants de manière connue, de manière à recueillir le 1,1-dichloro-1-fluoroéthane et le 1-chloro-1,1-difluoroéthane, les réactifs non convertis (chlorure de vinylidène et fluorure d'hydrogène) pouvant ensuite être recyclés au réacteur d'hydrofluoration. Dans une variante classique du procédé schématisé à la figure 1, une partie de la phase liquide (4) est vaporisée dans un bouilleur et renvoyée sous forme vapeur dans la colonne (1).

Ce premier mode d'application illustre que le procédé selon l'invention s'avère particulièrement intéressant lorsque le fluorure d'hydrogène utilisé comme réactif est mis en oeuvre en quantité excédentaire par rapport à la quantité stoechiométrique et que, pour des raisons évidentes d'économie, on souhaite recycler ce fluorure d'hydrogène excédentaire sous forme anhydre. Le procédé conforme à l'invention, qui vient d'être décrit en référence à la figure 1, peut s'appliquer à la production sélective de 1,1-dichloro-1-fluoroéthane ou de 1-chloro-1,1-difluoroéthane.

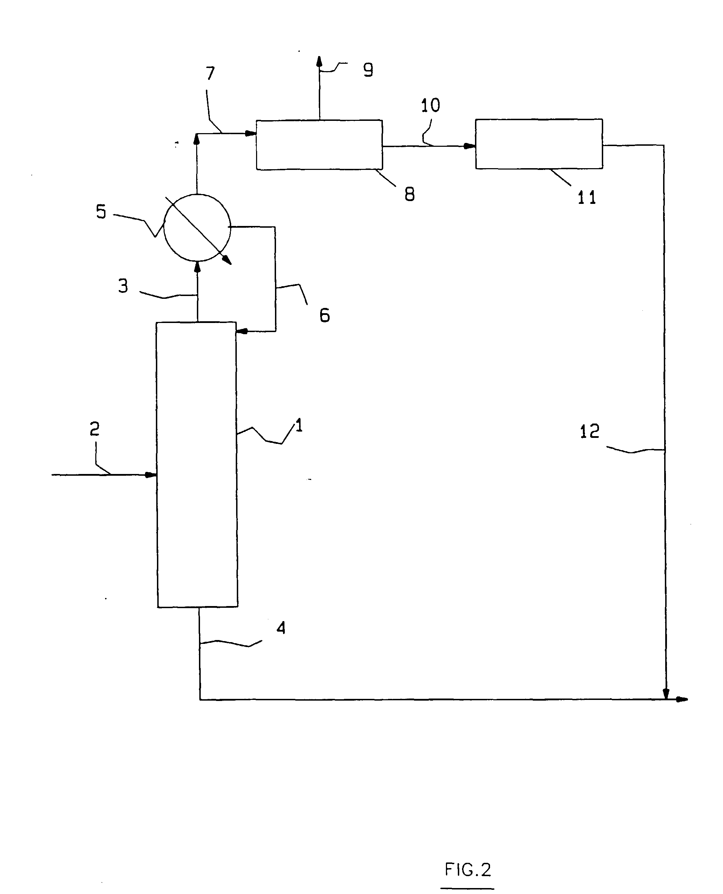

Le procédé schématisé à la figure 2 concerne la purification du 1,1-dichloro-1-fluoroéthane par chloration des hydrocarbures insaturés. A cet effet, on traite par du chlore, dans un réacteur de chloration non représenté, un mélange de produits réactionnels issu d'un réacteur d'hydrofluoration du chlorure de vinylidène contenant des hydrocarbures insaturés. Le mélange de produits traité, issu du réacteur de chloration contient typiquement de 75 à 99 % molaire de 1,1-dichloro-1-fluoroéthane, de 0,01 à 20 % molaire de chlore, de 0,01 à 5 % molaire d'impuretés chlorées et de 0,1 à 5 % molaire de chlorure d'hydrogène, ainsi que de 20 à 50 ppm en poids d'eau. Ce mélange réactionnel, désigné par la notation de référence (2), est introduit dans une colonne de distillation (1). La colonne de distillation (1) est maintenue à une pression de 1 à 10 bar et on ajuste la température en tête de la colonne (1) de manière à ce que le distillat quittant la colonne (1) par le conduit (3) contienne du 1,1-dichloro-1-fluoroéthane. Pour éviter toute formation d'hydrates de chlore solides dans le condenseur (5), une teneur massique en 1,1-dichloro-1-fluoroéthane de 3 à 50 % dans le distillat (3) est suffisante. Le distillat (3) comprend principalement le chlore, le chlorure d'hydrogène, l'azéotrope eau/1,1-dichloro-1-fluoroéthane et un peu de 1,1-dichloro-1-fluoroéthane excédentaire. Après condensation partielle dans le condenseur (5), la partie condensée du distillat (6) est renvoyée à la colonne comme reflux et la partie non condensée du distillat (7) est envoyée dans une unité de séparation (8), où le chlore et le chlorure d'hydrogène sont séparés du distillat, par exemple par distillation et sont évacués par le conduit (9). Le flux gazeux résiduel (10) contenant principalement du 1,1-dichloro-1-fluoroéthane et de l'eau est ensuite amené dans un sécheur (11) pour éliminer l'eau. Dans le sécheur (11), le flux gazeux (10) peut par exemple traverser des lits de matière adsorbante. Le gaz (12), recueilli à la sortie du sécheur (11) est constitué quasi exclusivement de 1,1-dichloro-1-fluoroéthane. Débarrassé de l'eau qu'il a entraînée hors du mélange de produits réactionnels, le 1,1-dichloro-1-fluoroéthane peut alors être mélangé à la phase liquide (4), recueillie au pied de la colonne de distillation. Cette phase liquide (4) contient principalement le 1,1-dichloro-1-fluoroéthane et les produits de chloration des hydrocarbures insaturés chlorés dans le réacteur de chloration. Cette phase liquide (4) peut être fractionnée en ses différents constituants de manière connue, par exemple par distillation, de manière à recueillir le 1,1-dichloro-1-fluoroéthane sous une forme substantiellement pure. Dans une variante classique du procédé schématisé à la figure 2, une partie de la phase liquide (4) est vaporisée dans un bouilleur et renvoyée sous forme vapeur dans la colonne (1).

Ce second mode d'application illustre que le procédé selon l'invention permet d'éviter toute formation d'hydrates entre le chlore et l'eau et évite donc leur accumulation dans les zones froides des installations de production d'hydrofluoroalcanes, notamment dans le condenseur en tête d'une colonne de distillation destinée à éliminer le chlore non réagi d'un mélange de produits réactionnels traité par du chlore.

## Revendications

1. Procédé d'élimination d'eau d'une solution comprenant de l'eau et au moins un composé liquide autre que de l'eau, par distillation azéotropique d'un azéotrope eau/entraîneur,
caractérisé en ce que l'entraîneur est sélectionné parmi le 1,1-dichloro-1-fluoroéthane, le 1-chloro-1,1-difluoroéthane, le 1,1-difluoroéthane, le 1,1,1-trifluoroéthane, le 1,1,1,2-tétrafluoroéthane et le trifluoroéthylène.

2. Procédé selon la revendication 1, caractérisé en ce que le composé liquide autre que de l'eau compris dans la solution est un hydrofluoroalcane.

3. Procédé selon la revendication 1 ou 2, caractérisé en ce que le composé liquide autre que de l'eau compris dans la solution constitue, en partie au moins, l'entraîneur.

4. Procédé selon une quelconque des revendications 1 à 3, caractérisé en ce que la solution est une composition de nettoyage, de dégraisssage ou de séchage d'objets.

5. Procédé selon une quelconque des revendications 1 à 3, caractérisé en ce que la solution est un mélange de produits réactionnels obtenu lors de la production d'un hydrofluoroalcane.

6. Procédé de production d'un hydrofluoroalcane dans lequel on recueille un mélange de produits réactionnels comprenant, en solution, l'hydrofluoroalcane et de l'eau, on soumet ledit mélange à une distillation azéotropique en présence d'un entraîneur formant un azéotrope avec l'eau, et on recueille de la distillation un distillat contenant l'azéotrope et une phase liquide contenant l'hydrofluoroalcane, caractérisé en ce qu'on sélectionne l'entraîneur parmi le 1,1-dichloro-1-fluoroéthane, le 1-chloro-1,1-difluoroéthane, le 1,1-difluoroéthane, le 1,1,1-trifluoroéthane, le 1,1,1,2-tétrafluoroéthane et le trifluoroéthylène.

7. Procédé selon la revendication 6, caractérisé en ce qu'on réalise la distillation en présence de 1 à 10 fois la quantité d'entraîneur strictement nécessaire pour former l'azéotrope.

8. Procédé selon la revendication 6 ou 7, caractérisé en ce que l'entraîneur est un constituant du mélange.

9. Procédé selon la revendication 8, caractérisé en ce que l'entraîneur est l'hydrofluoroalcane.

10. Procédé selon la revendication 6 ou 7, caractérisé en ce qu'on ajoute l'entraîneur au mélange.

11. Procédé selon une quelconque des revendications 6 à 10, caractérisé en ce que l'hydrofluoroalcane est le 1,1-dichloro-1-fluoroéthane.

## Patentansprüche

1. Verfahren zur Entfernung von Wasser aus einer Lösung, die Wasser und wenigstens eine von Wasser verschiedene flüssige Verbindung umfaßt, durch azeotrope Destillation eines Wasser/Schleppmittel-Azeotrops, dadurch gekennzeichnet, daß das Schleppmittel unter 1,1-Dichlor-1-fluorethan, 1-Chlor-1,1-difluorethan, 1,1-Difluorethan, 1,1,1-Trifluorethan, 1,1,1,2-Tetrafluorethan und Trifluorethylen ausgewählt ist.

2. Verfahren gemäß Anspruch 1, dadurch gekennzeichnet, daß die von Wasser verschiedene flüssige Verbindung, die in der Lösung enthalten ist, ein Fluorkohlenwasserstoff ist.

3. Verfahren gemäß Anspruch 1 oder 2, dadurch gekennzeichnet, daß die von Wasser verschiedene flüssige Verbindung, die in der Lösung enthalten ist, wenigstens zum Teil das Schleppmittel bildet.

4. Verfahren gemäß einem der Ansprüche 1 bis 3, dadurch gekennzeichnet, daß die Lösung eine Zusammensetzung zum Reinigen, Entfetten oder Trocknen von Gegenständen ist.

5. Verfahren gemäß einem der Ansprüche 1 bis 3, dadurch gekennzeichnet, daß die Lösung ein Gemisch von Reaktionsprodukten ist, das bei der Herstellung eines Fluorkohlenwasserstoffs erhalten wird.

6. Verfahren zur Herstellung eines Fluorkohlenwasserstoffs, bei dem man ein Gemisch von Reaktionsprodukten gewinnt, das den Fluorkohlenwasserstoff und Wasser in Lösung umfaßt, man besagtes Gemisch einer azeotropen Destillation in Gegenwart eines Schleppmittels, das mit Wasser ein Azeotrop bildet, unterwirft, und man aus der Destillation ein Destillat, das das Azeotrop enthält, und eine flüssige Phase, die den Fluorkohlenwasserstoff enthält, gewinnt, dadurch gekennzeichnet, daß man das Schleppmittel unter 1,1-Dichlor-1-fluorethan, 1-Chlor-1,1-difluorethan, 1,1-Difluorethan, 1,1,1-Trifluorethan, 1,1,1,2-Tetrafluorethan und Trifluorethylen auswählt.

7. Verfahren gemäß Anspruch 6, dadurch gekennzeichnet, daß man die Destillation in Gegenwart der 1 bis 10 fachen Menge an Schleppmittel, die genau zur Bildung des Azeotrops notwendig ist, durchführt.

8. Verfahren gemäß Anspruch 6 oder 7, dadurch gekennzeichnet, daß das Schleppmittel ein Bestandteil des Gemischs ist.

9. Verfahren gemäß Anspruch 8, dadurch gekennzeichnet, daß das Schleppmittel der Fluorkohlenwasserstoff ist.

10. Verfahren gemäß Anspruch 6 oder 7, dadurch gekennzeichnet, daß man das Schleppmittel zu dem Gemisch hinzufügt.

11. Verfahren gemäß einem der Ansprüche 6 bis 10, dadurch gekennzeichnet, daß der Fluorkohlenwasserstoff 1,1-Dichlor-1-fluorethan ist.

## Claims

1. Process for the removal of water from a solution comprising water and at least one liquid compound other than water by azeotropic distillation of a water/ entrainer azeotrope, characterized in that the entrainer is selected from 1,1-dichloro-1-fluoroethane, 1-chloro-1,1-difluoroethane, 1,1-difluoroethane, 1,1,1-trifluoroethane, 1,1,1,2-tetrafluoroethane and trifluoroethylene.

2. Process according to Claim 1, characterized in that the liquid compound other than water contained in the solution is a hydrofluoroalkane.

3. Process according to Claim 1 or 2, characterized in that the liquid compound other than water contained in the solution constitutes, at least in part, the entrainer.

4. Process according to any one of Claims 1 to 3, characterized in that the solution is a composition for cleaning, degreasing or drying articles.

5. Process according to any one of Claims 1 to 3, characterized in that the solution is a mixture of reaction products obtained during the production of a hydrofluoroalkane.

6. Process for the production of a hydrofluoroalkane in which there is recovered a mixture of reaction products comprising, in solution, the hydrofluoroalkane and water, the said mixture is subjected to azeotropic distillation in the presence of an entrainer which forms an azeotrope with water, and a distillate containing the azeotrope and a liquid phase containing the hydrofluoroalkane are recovered from the distillation, characterized in that the entrainer is selected from 1,1-dichloro-1-fluoroethane, 1-chloro-1,1-difluoroethane, 1,1-difluoroethane, 1,1,1-trifluoroethane, 1,1,1,2-tetrafluoroethane and trifluoroethylene.

7. Process according to Claim 6, characterized in that the distillation is carried out in the presence of 1 to 10 times the amount of entrainer which is strictly necessary to form the azeotrope.

8. Process according to Claim 6 or 7, characterized in that the entrainer is a constituent of the mixture.

9. Process according to Claim 8, characterized in that the entrainer is the hydrofluoroalkane.

10. Process according to Claim 6 or 7, characterized in that the entrainer is added to the mixture.

11. Process according to any one of Claims 6 to 10, characterized in that the hydrofluoroalkane is 1,1-dichloro-1-fluoroethane.
